# EUROPEAN PATENT SPECIFICATION

(11) **EP 2 108 367 B1**
(45) Date of publication and mention of the grant of the patent: **31.05.2017**
(21) Application number: 07850505.4
(22) Date of filing: 12.12.2007
(51) Int. Cl.: A23L 33/175, A61K 31/198, A61P 21/00, A61P 43/00

(54) **COMPOSITION FOR AMELIORATION/PREVENTION OF ADVERSE SIDE EFFECT IN STEROID THERAPY**
ZUSAMMENSETZUNG ZUR LINDERUNG/PRÄVENTION VON UNERWÜNSCHTEN NEBENWIRKUNGEN DER STEROIDTHERAPIE
COMPOSITION POUR ATTÉNUER/PRÉVENIR UN EFFET SECONDAIRE NUISIBLE DANS UNE THÉRAPIE AVEC DES STÉROÏDES

(30) Priority: 12.12.2006 JP 2006335057; 12.12.2006 JP 2006335059
(43) Date of publication of application: 14.10.2009
(60) Divisional application: 13159694.2
(73) Proprietor: EA Pharma Co., Ltd., Tokyo 104-0042 (JP)
(72) Inventor: NISHITANI, Shinobu, Kawasaki-shi Kanagawa 210-8681 (JP); TAKEHANA, Kenji, Kawasaki-shi Kanagawa 210-8681 (JP)
(74) Representative: Nicholls, Kathryn Margaret
(86) International application number: PCT/JP2007/073954
(87) International publication number: WO 2008/072663

(56) References cited:
- WO-A1-2004/058243
- WO-A1-2005/055997
- WO-A1-2005/094813
- JP-A- 07 118 150
- JP-A- 2000 026 289
- JP-A- 2000 026 290
- JP-A- 2005 289 928
- US-A1- 2005 277 624
- IMAJO ET AL: "Changes in BNP Levels and Relevant Symptoms after Administration of BCAA in Patients with Chronic Heart Failure Refractory to Treatment" JOURNAL OF CARDIAL FAILURE, CHURCHILL LIVINGSTONE, NAPERVILLE, IL, US, vol. 12, no. 8, 1 October 2006 (2006-10-01), page S186, XP005674404 ISSN: 1071-9164
- KOBAYASHI H. ET AL.: 'Modulations of muscle protein metabolism by branched-chain amino acids in normal and muscle-atrophying rats' THE JOURNAL OF NUTRITION vol. 136, no. 1, SUPPL., January 2006, pages 234S - 236S, XP008112428
- KOZIRIS L.P. ET AL.: 'Leucine as an antagonist of glucocorticoid-induced muscle atrophy' MEDICINE AND SCIENCE IN SPORTS AND EXERCISE vol. 29, no. 5, SUPPL., 1997, page S227 + ABSTR. NO. 1300, XP008112812
- SACHECK J.M. ET AL.: 'IGF-I stimulates muscle growth by suppressing protein breakdown and expression of atrophy-related ubiquitin ligases, atrogin-1 and MuRF1' AMERICAN JOURNAL OF PHYSIOLOGY, ENDOCRINOLOGY AND METABOLISM vol. 287, no. 4, 2004, pages E591 - E601
- KITAHARA Y. ET AL.: 'Effect of dietary supplementation with branched-chain amino acids on spontaneous motor activity and muscle function in beta.beta'-iminodipropionitrile-treated rats: a model for motorneuropathy' PHYSIOLOGY AND BEHAVIOR vol. 62, no. 3, 1997, pages 453 - 462, XP008112426
- DANIEL J. HAM AND AL.: "Leucine as a treatment for muscle wasting: a critical review", CLINICAL NUTRITION, 2014, pages 937-945,
- D. FREYSSENET AND AL.: "Effect of a 6 week endurance training programme and braanched -chain amino acid supplementation on histomorphometric characteristics of aged human muscle", ARCHIVES OF PHYSIOLOGY AND BIOCHEMISTRY, 1996, pages 157-162,
- J.M. SACHECK AND AL.: "Rapid disuse and denervation atrophy involve transcriptional changes similar to those of muscle wasting during systemic diseases", FASEB, 2007, pages 140-155,
- S. VERHOEVEN AND AL.: "Long-term leucine supplementation does not increase muscle mass or strength in healthy elderly men", AM J CLIN NUTR, 2009, pages 1468-1475,

### SEQUENCE LISTING

<110> EA Pharma Co., Ltd.
<120> COMPOSITION FOR AMELIORATION/PREVENTION OF ADVERSE SIDE EFFECT IN STEROID THERAPY
<130> 091142
<150> JP 2006-335057
   <151> 2006-12-12
<150> JP 2006-335059
   <151> 2006-12-12
<160> 16
<170> PatentIn version 3.3
<210> 1
   <211> 20
   <212> DNA
   <213> Artificial
<220>
   <223> 5'-primer of atrogin-1
<400> 1
   agcgcttctt ggatgagaaa 20
<210> 2
   <211> 20
   <212> DNA
   <213> Artificial
<220>
   <223> 3'-primer of atrogin-1
<400> 2
   tcttggctgc aacatcgtag 20
C210> 3
<211> 20
   <212> DNA
   <213> Artificial
<220>
   <223> 5'-primer of MuRF-1
<400> 3
   ccaggtgaag gaggaactga 20
<210> 4
   <211> 20
   <212> DNA
   <213> Artificial
<220>
   <223> 3'-primer of MuRF-1
<400> 4
   ctcctgctcc tgagtgatcc 20
<210> 5
   <211> 20
   <212> DNA
   <213> Artificial
<220>
   <223> 5'-primer of Myostatin
<400> 5
   acgctaccac ggaaacaatc 20
<210> 6
   <211> 20
   <212> DNA
   <213> Artificial
<220>
   <223> 3'-primer of Myostatin
<400> 6
   ggagtcttga cggctctcag 20
<210> 7
   <211> 21
   <212> DNA
   <213> Artificial
<220>
   <223> 5'-primer of Foxo 1
<400> 7
   cggccaatcc agcatgagcc c 21
<210> 8
   <211> 22
   <212> DNA
   <213> Artificial
<220>
   <223> 3'-primer of Foxo 1
<400> 8
   gtggggagga gagtcagaag tc 22
<210> 9
   <211> 22
   <212> DNA
   <213> Artificial
<220>
   <223> 5'-primer of Foxo 3a
<400> 9
   gctccaccac cagcaccaaa cc 22
<210> 10
   <211> 23
   <212> DNA
   <213> Artificial
<220>
   <223> 3'-primer of Foxo 3a
<400> 10
   cgagagggtt tgcatagact ggc 23
<210> 11
   <211> 24
   <212> DNA
   <213> Artificial
<220>
   <223> 5'-primer of Foxo 4
<400> 11
   gccatgacag aatgcctcag gatc 24
<210> 12
   <211> 24
   <212> DNA
   <213> Artificial
<220>
   <223> 3'-primer of Foxo 4
<400> 12
   ggctcaaagt tgaagtccag tccc 24
<210> 13
   <211> 20
   <212> DNA
   <213> Artificial
<220>
   <223> 5'-primer of REDD1
<400> 13
   tagtgcccac ctttcagttg 20
<210> 14
   <211> 20
   <212> DNA
   <213> Artificial
<220>
   <223> 3'-primer of REDD1
*<400> 14*
   gtcagggact ggctgtaacc 20
<210> 15
   <211> 21
   <212> DNA
   <213> Artificial
<220>
   <223> 5'-primer of KLF15
<400> 15
   ctgcagcaag atgtacacca a 21
<210> 16
   <211> 21
   <212> CDNA
   <213> Artificial
<220>
   <223> 3'-primer of KLF15
<400> 16
   tcatctgagc gtgaaaacct c 21

## Description

### Technical Field

The present invention relates to a composition for use in improving or suppressing side effects in steroid therapy, a composition for use in suppressing muscular atrophy-related gene expression and a combined use of the composition and a steroid drug. More particularly, the present invention relates to novel use of branched chain amino acid.

### Background Art

Symptomatic therapies of side effects in a steroid treatment currently include (1) infections: administration of antibacterial agent, (2) diabetes: administration of insulin and oral antidiabetic, (3) gastrointestinal tract symptom: administration of antiacids and H2 blocker, (4) osteoporosis: administration of vitamin D and calcium, (5) glaucoma: administration of ocular hypotensive agent, and (6) mental disorders and state of depression: administration of antipsychotic drug and the like. However, the only method of improving or suppressing myopathy, one of the severe side effects, is dose reduction of steroid. In addition, when plural side effects appear, a symptomatic medicament for each symptom should be taken to suppress the symptom. Furthermore, steroid dependency where steroid side effects are suppressed by other steroid and the like also pose problems.

Although a possibility of dose reduction of steroid by branched chain amino acid in the treatment of chronic rheumatoid arthritis has been suggested (patent document 1), it is not described that the branched chain amino acid suppresses side effect itself of steroid drug. For steroid drugs, even though the dose could be reduced, a long-term use of a small amount of steroid is considered to cause side effects of the same level as those caused by a high dose thereof. It is therefore considered difficult to mitigate the side effects of steroid drug only by dose reduction.

On the contrary, use of a small amount of steroid drug out of fear of side effects may not afford a sufficient treatment effect. A specific example thereof is a pulse therapy using a large amount of a steroid drug. This method requires a large amount of a steroid drug to rapidly suppress symptoms such as lethal symptom, severe organ disorder, symptoms in an active disease period and the like, where injudicious dose reduction involves risk. In other words, dose reduction of steroid may be life-threatening for patients. Accordingly, there is a demand for a method capable of suppressing side effects of steroid drug without requiring dose reduction.

One of known side effects of steroid therapy is muscular atrophy action. Recent reports have documented that such muscular atrophy action is caused by promoted expression of transcription of atrogin-1 gene and myostatin gene due to activation of transcription of these gene caused by dephosphorylation of transcription factor Foxo resulting from inhibition of the activity of Akt1 (protein kinase B) by steroid (glucocorticoid) (non-patent documents 1 - 3). The atrogin-1 gene and MuRF-1 gene are genes encoding ubiquitinligase, namely, proteasomal degradation inducing genes, which are also called muscular atrophy genes (Atrogene). In addition, myostatin gene belongs to the TGFβ family, and is known to be a negative regulator for the growth of muscle (non-patent document 4). Furthermore, FOXO gene is reported to show promoted expression when muscular atrophy occurs (non-patent documents 5 and 6). In addition, KLF15 and REDD1 genes are known to be "metabolism·nutrition regulation-related genes" relating to the metabolism or malnutrition considered to occur simultaneously or along with muscular atrophy (non-patent documents 7 and 8)

There are known some diseases where pathology is aggravated (muscular atrophy and cancer cachexia) by, besides side effects of steroid, increased expression of muscular atrophy gene (non-patent document 9). Examples of such disease include diabetes, cancer, renal failure, cardiac failure, AIDS, cirrhosis, various inflammatory diseases, malnutrition disease and the like. Muscular atrophy is a serious problem that degrades the QOL of patients. Accordingly, there is a demand for a drug capable of suppressing muscular atrophy due to administration of steroid drug or the above-mentioned diseases.

It is known that IGF-1 (Insulin-like Growth Factor)/PI3K (phosphoinositol 3-kinase)/AKT/mTOR (mammalian Target of Rapamycin) pathway plays an important role in the expression of muscular atrophy gene. When this pathway is suppressed for some reason, the expression of muscular atrophy gene is considered to increase and cause muscle atrophy (non-patent documents 10 and 11). It is reported that administration of dexamethasone to rat decreases feed intake and body weight, causing muscular atrophy, and they are suppressed by IGF-1 (non-patent document 12). On the other hand, IGF-1 is reported to suppress expression of atrogin-1 and MuRF-1 induced by dexamethasone in muscle cells (non-patent documents 10 and 13). In addition, it is shown that muscular atrophy genes (atrogin-1 or MuRF-1) knock-out mice are free of muscular atrophy (non-patent document 2).

In the meantime, the relationship between branched chain amino acid (isoleucine, leucine and valine) and suppressive action on muscular atrophy has been reported (non-patent documents 14 and 15). However, the report discusses muscular atrophy in view of the effect on protein degradation rate and synthesis rate. On the other hand, some describe that only an insufficient effect for suppression of protein degradation in muscular atrophy, particularly muscle, is afforded with branched chain amino acid alone, and a method of determining the efficiency and effect thereof remains unresolved (non-patent documents 15, 16, 17 and 18). Branched chain amino acids including leucine are known to promote protein synthesis by activating mTOR (non-patent document 19). However, its effect on the expression of muscular atrophy gene is not known. Moreover, genes whose expression in myoblast cell line C2C12 changes due to IGF-1 tend to be inversely regulated by the co-presence of an mTOR inhibitor, Rapamycin, and known to be among them is MuRF-1, one of the muscular atrophy genes (non-patent document 20). However, it is not known that branched chain amino acids (e.g., leucine etc.) suppress expression of muscular atrophy genes (atrogin-1 and MuRF-1) that increases due to the stimulation inducing muscular atrophy.
patent document 1: WO2005/055997
non-patent document 1: Proc. Natl. Acad. Sci. U.S.A., 98: 14440-14445 (2001)
non-patent document 2: Science, 294: 1704-1708 (2001) non-patent document 3: Nature Med., Vol. 10, (6): 584-585 (2004)
non-patent document 4: Curr Opin Pharmacol., 7 (3):310-5 (2007)
non-patent document 5: J. Biol. Chem., Vol. 279, (39):4114-41123(2004)
non-patent document 6: J. Biol. Chem., Vol. 282, (29):21176-21186(2007)
non-patent document 7: BBRC Vol. 327:920-926(2005)
non-patent document 8: J. Biol. Chem., Vol. 281,(51):39128-39134(2006)
non-patent document 9: FASEB J., 18: 39-51 (2004)
non-patent document 10: Molecular Cell, Vol. 14, 395-403 (2004)
non-patent document 11: Cell, Vol. 117, 399-412 (2004)
non-patent document 12: Endocrinology 146: 1789-1797 (2005)
non-patent document 13: Am. J. Physiol. Endocrinol Metab., 287: E591-E601(2004)
non-patent document 14: J. Nutr., 136(1 Suppl):234S-6S (2006)
non-patent document 15: J. Nutr., 136(1 Suppl):237S-42S (2006)
non-patent document 16: J. Nutr., 136(1 Suppl):264S-68S (2006)
non-patent document 17: J. Nutr., 136(1 Suppl):308S-13S(2006)
non-patent document 18: J. Nutr.,;136(1 Suppl):314S-18S (2006)
non-patent document 19: J. Nutr., 2006 Jan;136(1 Suppl):269S-73S
non-patent document 20: J. Biol. Chem., Vol. 280, No.4: 2737-2744 (2005)

### Disclosure of the Invention

### Problems to be Solved by the Invention

Steroid drugs are very effective pharmaceutical products for various symptoms such as collagen disease. However, use of a steroid drug in a high dose or for a long time causes severe side effects that could sometimes be lethal. An object of the present invention is to provide a means of improving or suppressing side effects caused by steroid treatments.

Another object of the present invention is to provide a means capable of suppressing muscular atrophy occurring in association with the progression of various diseases, and highly influential on reduced QOL of patients.

### Means of Solving the Problems

The present inventors have conducted intensive studies in an attempt to solve the aforementioned problems and found that three kinds of branched chain amino acids of isoleucine,
leucine and valine (hereinafter to be also referred to as BCAA) are effective for use in improving side effects caused by the administration of steroid drugs, such as muscular atrophy, decreased muscle function, muscular pain, arthritic pain, impaired glucose tolerance, decreased bone metabolism, impaired immunity, loss of appetite, fatigability, body weight loss and for suppressing muscular atrophy associated with promoted expression of muscular atrophy-related gene, which resulted in the completion of the present invention.

Accordingly, the present invention provides the following.
[1] A composition for use in improving or suppressing a side effect in a steroid treatment, comprising isoleucine, leucine and valine as active ingredients wherein the side effect is at least one kind selected from the group consisting of muscular atrophy, decreased muscle function, muscular pain, arthritic pain, impaired glucose tolerance, loss of appetite, body weight loss, decreased bone metabolism, impaired immunity and fatigability.
[2] The composition for use according to [1], wherein the side effect is impaired glucose tolerance and/or decreased bone metabolism.
[3] The composition for use according to [1], wherein muscular atrophy shows promoted expression of muscular atrophy-related gene.
[4] A composition for use in suppressing muscular atrophy associated with promoted expression of muscular atrophy-related gene, comprising isoleucine, leucine and valine as active ingredients, wherein the promoted expression is associated with renal failure, diabetes, lung failure, liver failure or cardiac failure.
[5] The composition for use according to [4] wherein the muscular atrophy-related gene is at least one kind selected from the group consisting of atrogin-1 gene, MuRF-1 gene, myostatin gene, FOXO1 gene, FOXO3a gene, FOXO4 gene, REDD1 gene and KLF15 gene.
[6] The composition for use according to any one of [1] to [5], comprising not less than 1 g of isoleucine, leucine and valine in one-time ingestion.
[7] The composition for use according to any one of [1] to [6], wherein a weight ratio of isoleucine, leucine and valine is 1:1.5 - 2.5:0.8 - 1.7.
[8] The composition for use according to any one of [1] to [7], for use in combination with a steroid drug.
[9] The composition for use according to any one of [1] to [7], further comprising a steroid drug.
[10] The composition for use according to any one of [1] to [9], which is a medicament.
[11] The composition for use according to any one of [1] to [9], which is a food, or dietary supplement.
[12] The composition for use according to [11], wherein the food is a concentrated liquid diet.
[13] Use of isoleucine, leucine and valine for the production of a composition for use according to any one of [1] to [12].

### Effect of the Invention

A composition comprising three kinds of branched chain amino acids of isoleucine, leucine and valine as active ingredients, which is provided by the present invention, is for use in improving or suppressing at least one of the following side effects of steroid treatments: such as muscular atrophy, decreased muscle function, muscular pain, arthritic pain, impaired glucose tolerance, decreased bone metabolism, impaired immunity, loss of appetite, fatigability, and body weight loss.

The composition for use in suppressing muscular atrohpy associated with promoted expression of muscular atrophy-related gene of the present invention can directly and effectively improve a muscular atrophy state by suppressing the expression of muscular atrophy-related gene. Particularly, the composition of the present invention is useful for the prophylaxis or treatment of muscular atrophy which is a side effect of a steroid treatment and muscular atrophy associated with renal failure, and useful for the prophylaxis or treatment of muscular atrophy associated with various other diseases characterized by increased expression of Atrogin-1 and MuRF-1 genes.

In addition, the composition for use in suppressing muscular atrophy associated with promoted expression of muscular atrophy-related gene of the present invention can be used for the prophylaxis or treatment of various chronic or acute diseases whose pathology is aggravated by muscular atrophy due to promoted expression of muscular atrophy-related gene. Such diseases are characterized by undesired decrease of body weight, specifically a decreased ability to exercise associated with atrophy of skeletal muscles. Using the composition of the present invention, a decreased ability to exercise due to a decreased amount of muscle can be prevented or treated. As a result, falling of patients and bedridden patients can be prevented, and hospitalization period and treatment period are expected to be shortened.

In addition, since the medicament of the present invention comprises branched chain amino acids as active ingredients, it is highly safe and hardly causes side effects. Therefore, the medicament is useful as a pharmaceutical product for the treatment or prophylaxis of muscular atrophy as side effect of a steroid treatment and muscular atrophy associated with various other diseases. Furthermore, since the three kinds of branched chain amino acids of isoleucine, leucine and valine contained in the composition of the present invention have established safety, the composition of the present invention is highly safe and can be used not only for pharmaceutical use but also for food.

### Brief Description of the Drawings

Fig. 1 shows body weight profiles and changes in feed intake in the BCAA administration group and the vehicle administration group.
Fig. 2 shows comparison of the muscle weights of gastrocnemius muscle and soleus muscle among the BCAA administration group, the vehicle administration group and the control (normal group).
Fig. 3 shows comparison of grip strength measurement values among the BCAA administration group, the vehicle administration group and the control (normal group). Student t-test *p<0.05
Fig. 4 shows comparison of blood glucose levels among the BCAA administration group, the vehicle administration group and the control (normal group).
Fig. 5 shows comparison of plasma insulin levels among the BCAA administration group, the vehicle administration group and the control (normal group).
Fig. 6 shows comparison of the muscle weights of gastrocnemius muscle and soleus muscle at day 1 and day 2 of the recovery period (recover; R) between the BCAA administration group and the vehicle administration group.
Fig. 7 shows comparison of the plasma ALP levels between the BCAA administration group and the vehicle administration group. Student t-test *p<0.05
Fig. 8 shows body weight profiles and changes of feed intake in rats.
Fig. 9 shows comparison of the muscle weights of gastrocnemius muscle and soleus muscle.
Fig. 10 shows comparison of expressions among atrogin-1 gene, MuRF-1 gene and Myostatin gene, wherein the value of each group is relative to one value of the control group as 1.
Fig. 11 shows comparison of expressions among FOXO1, FOXO3a and FOXO4 genes, wherein the value of each group is relative to one value of the control group as 1.
Fig. 12 shows comparison of expressions of REDD1 gene, wherein the value of each group is relative to one value of the control group as 1.
Fig. 13 shows comparison of expressions of KLF15 gene, wherein the value of each group is relative to one value of the control group as 1.
Fig. 14 shows comparison of body weights and feed intake profiles at day 1 and day 2 of the recovery period (recover; R).
Fig. 15 shows comparison of the muscle weights of gastrocnemius muscle and soleus muscle at day 1 and day 2 of the recovery period (recover; R).
Fig. 16 shows comparison of expressions of atrogin-1 gene and MuRF-1 gene at day 1 and day 2 of the recovery period (recover; R), wherein the value of each group is relative to one value of the control group as 1.
Fig. 17 shows comparison of expressions of atrogin-1 gene and MuRF-1 gene in renal failure model rats, wherein the value of each group is relative to one value of the control group as 1.

### Best Mode for Carrying out the Invention

The compositions of the present invention for use in improving or suppressing a side effect in a steroid treatment wherein the side effect is at least one kind selected from the group consisting of muscular atrophy, decreased muscle function, muscular pain, arthritic pain, impared glucose tolerance, loss of appetite, body weight loss, decreased bone metabolism, impaired immunity and fatigability, or for use in suppressing muscular atrophy associated with promoted expression of muscular atrophy-related gene, wherein the promoted expression is associated with renal failure, diabetes, lung failure, liver failure or cardiac failure, are characterized by isoleucine, leucine and valine contained therein as active ingredients (hereinafter sometimes to be generally referred to as the composition of the present invention).

The composition of the present invention is useful for the improvement or suppression of side effects associated with a steroid treatment. In the present specification, the "improvement" includes "prophylaxis, mitigation and treatment", and means that the side effects associated with steroid treatments, i.e., "muscular atrophy", "decreased muscle function", "muscular pain", "arthritic pain", "impaired glucose tolerance", "decreased bone metabolism", "impaired immunity", "loss of appetite", "body weight loss", "fatigability" move in the direction of normalization, and further that the development of the side effects is prevented or suppressed in advance.

In a serious case, the "development of side effects associated with a steroid treatment" sometimes impairs QOL markedly due to the side effects of steroid drug, even though the drug is used for the treatment of the disease. In some cases, it is life-threatening. In the case of a hospitalized patient, for example, the symptoms may not be improved to the level permitting discharge from the hospital. In the case of a patient requiring nursing care or care, the symptoms may not be improved sufficiently enough to shorten the time of a nurse or care assistant, which is necessary for nursing care or care of the patient. However, the composition of the present invention is effective for both cases.

Examples of the side effects of steroid treatments include (1) aggravation of infections, concealment of induction and symptoms, impaired immunity, (2) adrenocortical insufficiency, (3) impaired glucose tolerance such as induction or aggravation of diabetes, elevation of blood glucose level and the like, (4) gastrointestinal tract ulcer, gastrointestinal hemorrhage, gastrointestinal perforation, hemorrhagic pancreatitis, (5) cramp, intracranial hypertension, (6) mental disorders, state of depression, (7) decreased bone metabolism such as osteoporosis (particularly, compression fracture of the spine) and the like, (8) aseptic necrosis of bone head (femoral, humerus bone fracture), (9) myopathy (loss of muscle strength accompanied by muscular atrophy, particularly, decreased muscle weight and loss of muscle strength of proximal muscle, decreased muscle function), body weight loss, (10) glaucoma, high eye pressure, posterior capsular cataract, (11) thrombus (hypercoagulability), (12) cardiac rupture due to myocardial infarction, (13) aggravation of asthma attack, (14) anaphylaxis due to injection, (15) moon-shaped face, buffalo hump, (16) elevation of blood pressure due to mineral effect, (17) sodium-water retention (edema) body weight increase, hypokalemic alkalosis, (18) development disorders in childhood, (19) menstrual disorder, (20) decrease in sperm motility and number, (21) acne, hypertrichosis, hair loss, deposit of pigment, (22) dermal thinning, weakening, subcutaneous congestion, linear purpura, facial erythema, panniculitis, (23) wound healing disorder, (24) symptom of irritation (eruption) itching sensation, hiccup, (25) euphoria, insomnia, headache, dizziness, (26) dyshidrosis, excessive urination, leukocytosis, (27) fatty liver, nitrogen imbalance, (28) GOT, GPT, ALP increase, (29) hyperlipidemia, hypercholesterolemia, steroidnephropathy, (30) nausea, vomiting, stomachache, heartburn, sense of abdominal fullness, dry mouth, diarrhea, excessive appetite, (31) retinal disorder or eyophthalmos due to central serous chorioretinopathy, (32) muscular pain, arthritic pain, fever, feeling of fatigue, (33) atrophy in topical tissue due to muscle, intradermal or subcutaneous injection, depression, body weight loss, (34) thrombus, phlebitis, pain, swelling, tender aggravation during intravenous injection, (35) withdrawal syndrome; systemic symptom: fever, headache, fatigability, generalized fatigability, feeling of weakness, shock/digestive organ:anorexia, loss of appetite, nausea and vomiting, diarrhea/nerve system: headache, anxiety, excitement/cramp, disturbance of consciousness, muscular pain, arthritic pain. The present invention is particularly effective for side effects such as "muscular atrophy", "decreased muscle function", "muscular pain", "arthritic pain", "impaired glucose tolerance", "decreased bone metabolism", "impaired immunity", "loss of appetite", "body weight loss", "fatigability".

Examples of the target disease of a steroid treatment include (1) endocrine diseases: chronic adrenocortical insufficiency (primary, secondary, pituitary, iatrogenic), acute adrenocortical insufficiency (adrenal crisis), adrenogenital syndrome, subacute thyroiditis, thyrotoxicosis [thyroidal (toxic) crisis], malignant exophthalmos associated with thyroidal disease, isolated ACTH deficiency, (2) rheumatism disease: chronic rheumatoid arthritis, juvenile rheumatoid arthritis (including Still's disease), rheumatic fever (including rheumatic pancarditis), polymyalgia rheumatica, (3) collagen disease: erythematosus (systemic and chronic discoidlupus), systemic vasculitis (including aortitis syndrome, periarteritis nodosa, polyarteritis, Wegener granulomatosis), polymyositis (dermatomyositis), scleroderma, (4) renal diseases: nephrosis and nephrosis syndrome, (5) cardiac disease: congestive heart failure, (6) allergic disease: bronchial asthma, asthmatic bronchitis (including childhood asthmatic bronchitis), allergy or intoxication due to medicament and other chemical substances (including drug eruption, toxic eruption), serum sickness (7) severe infections: severe infections (in combination with chemical therapy), (8) blood diseases: hemolytic anemia (with suspected immunity or immune mechanism), leukemia (acute leukemia, acute blastic crisis of chronic myelogenous leukemia, chronic lymphatic leukemia) (including skin leukemia), agranulocytosis (primary, secondary), purpura (thrombocytopenic and nonthrombocytopenic), aplastic anemia, hemorrhagic diathesis due to disorder of coagulation factor, (9) gastrointestinal diseases: localized enteritis, ulcerative colitis, (10) severe debilitating disease: improvement of general condition of severe debilitating disease (including cancerlate stage, sprue), (11) hepatic diseases: fulminant hepatitis (including clinically severe cases), cholestatic acute hepatitis, chronic hepatitis (active type, recrudescent acute type, cholestatic type) (limited to intractable one unresponsive to general treatments and with persistent noticeable abnormality in liver function), cirrhosis (active type, condition accompanying intractable ascites, condition accompanying cholestasis), (12) lung disease: sarcoidosis (excluding condition with bilateral hilar adenopathy alone), diffuse interstitial pneumonia (lung fibrosis) (including irradiation pneumonitis), (13) tuberculous disease (in combination with antitubercular agent), pulmonary tuberculosis (limited to miliary tuberculosis, severe tuberculosis), meningitis tuberculosa, tuberculous pleurisy, peritoneal tuberculosis, tuberculous pericarditis, (14) neurological disease: encephalomyelitis (including encephalitis, myelitis) (to be used for a short period for primary encephalitis, when intracranial hypertensive symptom is observed and other drugs provide insufficient effect), peripheral neuritis (including Guillain-Barre syndrome), myotonia, myasthenia gravis, multiple sclerosis (including optic myelitis), chorea minor, facial paralysis, spinal arachnoiditis, (15) malignant tumor: malignant lymphoma (lymphosarcomatosis, reticulosarcomatosis, Hodgkin's disease, cutaneous reticulosis, mycosis fungoides) and similar disease (related disease), eosinophilic granuloma, recurrence metastasis of breast cancer, (16) other medical diseases: idiopathic hypoglycemia, unexplained fever, (17) infections: SARS and the like. Furthermore, a steroid treatment and the like are also included, which are used for the purpose of suppressing rejection during organ transplantation such as liver transplantation, kidney transplantation.

Examples of the steroid drug to be used for a steroid treatment include hydrocortin, cortisone, prednisolone, methylprednisolone, triamcinolone, triamcinolone acetonide, paramethasone, dexamethasone, betamethasone, hexestrol, methimazole, fluocinonide, fluocinolone acetonide, fluorometholon, beclometasone dipropionate, estriol, diflorasone diacetate, diflucortolone valerate, difluprednate.

In the present invention, the "muscular atrophy-related gene" generally means "muscular atrophy gene" and "metabolism· nutrition regulation-related gene" relating to metabolism or malnutrition considered to occur simultaneously or in association with muscular atrophy.

The "muscular atrophy gene" refers to a gene showing different expression in the cell when the muscle is atrophied, as compared to the expression before being atrophied, and includes a gene showing promoted expression and a gene showing suppressed expression. Preferred is a gene showing promoted expression. Preferable specific examples thereof include ubiquitinligase gene, particularly, atrogin-1 gene, MuRF-1 gene and myostatin gene known to show promoted expression during muscular atrophy associated with glucocorticoid excess. Furthermore, preferable examples of the metabolism·nutrition regulation-related gene also include FOXO gene such as FOXO1 gene, FOXO3a gene, FOXO4 gene and the like, REDD1 gene and KLF15 gene.

In the present invention, the etiology of muscle atrophy is not limited to glucocorticoid excess, and any cause that induces expression variation of muscular atrophy-related gene is included in the etiology. In addition, the excess of glucocorticoid may be endogenous or exogenous.

In the present invention, the "suppression of muscular atrophy-related gene expression" means decreasing or increasing the expression of a gene that was promoted or suppressed, respectively, when the muscle was atrophied. It preferably means decreasing a promoted expression of a muscular atrophy-related gene.

The composition of the present invention preferably suppresses, via an expression suppressive action of muscular atrophy-related gene, muscular atrophy associated with promoted expression of muscular atrophy-related gene.

As the muscular atrophy-related gene whose promoted expression is suppressed by the composition of the present invention, the above-mentioned atrogin-1 gene, MuRF-1 gene, myostatin gene, FOXO1 gene, FOXO3a gene, FOXO4 gene, REDD1 gene, KLF15 gene and the like are preferable, and atrogin-1 gene, MuRF-1 gene, myostatin gene and the like are more preferable.

The atrogin-1 gene, MuRF-1 gene, myostatin gene, FOXO1 gene, FOXO3a gene, FOXO4 gene, REDD1 gene and KLF15 are known, and the sequences thereof are disclosed in the NCBI database. For example, in the NCBI gene transcript database UniGene (http://www.ncbi.nlm.nih.gov/entrez/query.fcgi?db=unigene), the atrogin-1 gene is a gene whose transcripts in mouse, rat and human can be defined by IDs of Mm.292042, Rn.72619, Hs.403933, respectively, and the like and, particularly in human, it is defined as *606604 F-BOX ONLY PROTEIN 32; FBX032 in Online Mendelian Inheritance in Man^{™} (OMIM^{™}) .

MuRF-1 gene is a gene whose transcripts in mouse, rat and human can be defined by IDs of Mm.261690 or Mm.331961, Rn.40636, Hs.279709 and the like in UniGene and, particularly in human, it is defined as *606131 RING FINGER PROTEIN 28; RNF28 in OMIM^{™}.

Myostatin gene is a gene whose transcripts in mouse, rat and human can be defined by IDs of Mm.3514, Rn.44460, Hs.41565 and the like in UniGene and, particularly in human, it is defined as *603936 GROWTH/DIFFERENTIATION FACTOR 11; GDF11 in OMIM^{™}.

FOXO1 gene is a gene whose transcripts in mouse, rat and human can be defined by IDs of Mm.29891, Mm.395558, Rn.116108, Hs.370666 and the like in UniGene and, particularly in human, it is defined as *136533 FORKHEAD BOX 01A; FOXO1A in OMIM^{™}. FOXO3a gene is a gene whose transcripts in mouse, rat and human can be defined by IDs of Mm.296425, Rn.24593, Hs.220950 and the like in UniGene and, particularly in human, it is defined as *602681 FORKHEAD BOX O3A; FOXO3A in OMIM^{™}.

FOXO4 gene is a gene whose transcripts in mouse, rat and human can be defined by IDs of Mm.371606, Rn.19646, Hs.584654 and the like in UniGene and, particularly in human, it is defined as **300033 MYELOID/LYMPHOID OR MIXED LINEAGE LEUKEMIA, TRANSLOCATED TO, 7; MLLT7 in OMIM^{™}. REDD1 gene is a gene whose transcripts in mouse, rat and human can be defined by IDs of Mm.21697, Rn.9775, Hs.523012 and the like in UniGene and, particularly in human, it is defined as *607729 REGULATED IN DEVELOPMENT AND DNA DAMAGE RESPONSES 1 in OMIM^{™}.

KLF15 gene is a gene whose transcripts in mouse, rat and human can be defined by IDs of Mm.41389, Rn.22556, Hs.272215 and the like in UniGene and, particularly in human, it is defined as *606465 KRUPPEL-LIKE FACTOR 15; KLF15 in OMIM^{™}.

In the present invention, "muscular atrophy" means the condition where the muscles become thin and the muscular strength is weakened.

In one embodiment, "muscular atrophy" is associated with promoted expression of muscular atrophy gene in conjunction with endogenous or exogenous glucocorticoid excess. Specifically, (1) examples of muscular atrophy caused by steroid drug administration, which is a typical example of exogenous glucocorticoid excess, include muscular atrophy induced by steroid drug administered as a pharmaceutical product for the purpose of mitigation of symptoms such as various malignant tumors, cancer; respiratory diseases such as pneumonia, chronic obliterative pulmonary diseases, sarcoidosis, lung fibrosis; infections associated with debilitating inflammation such as AIDS (acquired immunodeficiency syndrome), virus hepatitis, influenza; sepsis associated with infections; autoimmune diseases such as chronic rheumatoid arthritis, IBD (inflammatory bowel disease), collagen disease; diabetes, renal failure, lung failure, liver failure, cardiac failure, (2) examples of endogenous glucocorticoid excess include muscular atrophy associated with Cushing's syndrome with characteristic clinical symptoms (e.g., hypercortisolemia with promoted adrenal function) caused by some unexplained reason including adrenal gland tumor, or hypercortisolemia induced as a result of biological response due to other chronic inflammations, and (3) decrease of muscle mass or decrease of muscle strength associated with anorexia, hypercatabolism due to excessive stress in terminal symptoms in general; muscular atrophy due to hospitalization, akinesia, bedridden state or weightless flight.

In another embodiment, the "muscular atrophy" only needs to be that associated with promoted expression of muscular atrophy gene, even if the causal association with glucocorticoid excess is not directly clear. Specific examples include muscular atrophy associated with various malignant tumors, cancer; respiratory diseases such as pneumonia, chronic obliterative pulmonary diseases, sarcoidosis, lung fibrosis; infections associated with debilitating inflammation such as AIDS (acquired immunodeficiency syndrome), virus hepatitis, influenza; sepsis associated with infections; autoimmune diseases such as chronic rheumatoid arthritis, IBD (inflammatory bowel disease), collagen disease; muscular atrophy associated with disorders or diseases such as diabetes, renal failure, lung failure, liver failure, cardiac failure; decrease of muscle mass or decrease of muscle strength associated with anorexia, hypercatabolism; muscular atrophy due to hospitalization, akinesia, bedridden state or weightless flight.

As the muscular atrophy associated with disorders or diseases other than glucocorticoid excess, muscular atrophy associated with renal failure is effective.

In the present specification, the "suppression" of muscular atrophy includes "preventing muscular atrophy from occurring, mitigating or treating" muscular atrophy, and specifically means bringing the muscle to the state before developing muscular atrophy by preventing the above-mentioned muscular atrophy from occurring, mitigating or treating the "muscular atrophy". When the muscle is hypertrophied after returning to the state before developing muscular atrophy by applying the present invention, such case is also encompassed in the context of the present invention.

Whether or not the expression of the muscular atrophy gene is promoted can be examined by a method known per se. For example, a method comprising harvesting, by biopsy, a cell, preferably a muscle cell (e.g., skeletal muscle cell, smooth muscle cell, cardiac muscle cell, etc.) predicted to express muscular atrophy gene, amplifying atrogin-1 gene or MuRF-1 gene by PCR, and detecting the gene can be mentioned.

Examples of exogenous glucocorticoid (steroid drug) include hydrocortin, cortisone, prednisolone, methylprednisolone, triamcinolone, triamcinolone acetonide, paramethasone, dexamethasone, betamethasone, hexestrol, methimazole, fluocinonide, fluocinolone acetonide, fluorometholon, beclometasone propionate, estriol, diflorasone diacetate, diflucortolone valerate, difluprednate.

As mentioned above, it has been reported that activated transcription of atrogin-1 gene and MuRF-1 gene results from dephosphorylation of transcription factor Foxo due to inhibition of the activity of Akt1 (protein kinase B) by glucocorticoid.

In the present invention, the "promoted expression of muscular atrophy-related gene" by etiology other than glucocorticoid excess means promoted expression of the above-mentioned "muscular atrophy-related gene" in muscular atrophy associated with various malignant tumors, cancer; respiratory diseases such as pneumonia, chronic obliterative pulmonary diseases, sarcoidosis, lung: fibrosis; infections associated with debilitating inflammation such as AIDS (acquired immunodeficiency syndrome), virus hepatitis, influenza; sepsis associated with infections; autoimmune diseases such as chronic rheumatoid arthritis, IBD (inflammatory bowel disease), collagen disease; disorders or diseases such as diabetes, renal failure, lung failure, liver failure, cardiac failure; decrease of muscle mass or decrease of muscle strength associated with anorexia, hypercatabolism; muscular atrophy due to hospitalization, akinesia, bedridden state or weightless flight.

Isoleucine, leucine and valine, which are active ingredients of the composition of the present invention, each may be in the form of any of L-form, D-form and DL-form when in use, preferably L-form or DL-form, more preferably L-form.

In addition, isoleucine, leucine and/or valine in the present invention each may be in the form of not only a free form but also a salt form when in use. Isoleucine, leucine and/or valine in the form of such salt are/is also encompassed in the present invention. Examples of the salt form include a salt with acid (acid addition salt), a salt with base (base addition salt) and the like. A pharmaceutically acceptable salt is preferably selected.

Examples of the acid to be added to isoleucine, leucine and/or valine to form a pharmaceutically acceptable acid addition salt include inorganic acids such as hydrogen chloride, hydrogen bromide, sulfuric acid, phosphoric acid and the like; organic acids such as acetic acid, lactic acid, citric acid, tartaric acid, maleic acid, fumaric acid, monomethylsulfuric acid.

Examples of the base to be added to isoleucine, leucine and/or valine to form a pharmaceutically acceptable base addition salt include metal hydroxide or metal carbonate such as sodium, potassium, calcium, or inorganic base such as ammonia; organic base such as ethylenediamine, propylenediamine, propylenediamine, ethanolamine, monoalkylethanolamine, dialkylethanolamine, diethanolamine, triethanolamine.

The content of the three kinds of branched chain amino acids of isoleucine, leucine and valine in the composition of the present invention for application to human is not less than 0.1 g, preferably not less than 1 g, in total for one-time ingestion. From the aspect of easy ingestion, the above-mentioned amount of one-time ingestion is preferably not more than 100 g, more preferably not more than 10 g.

In the present specification, the "amount of one-time ingestion" is the amount of active ingredients to be administered at once when the composition of the present invention is a medicament, and the amount of active ingredients to be ingested at once when the composition of the present invention is food. Particularly, in the case of food, the total amount of food to be ingested varies among individuals, and cannot be easily defined generally as the content of active ingredients in the composition. Therefore, it is recommended to define the amount as an amount for one-time ingestion of the active ingredients. Such amount for one-time ingestion varies depending on the age, body weight, sex, severity of muscular atrophy.

The composition of the present invention contains three kinds of branched chain amino acids of isoleucine, leucine and valine as active ingredients. The mixing ratio of the three kinds of amino acid in weight ratio is generally within the range of 1:1.5 - 2.5:0.8 - 1.7, particularly preferably 1:1.9 - 2.2:1.1 - 1.3. Outside these ranges, an effective action and effect is difficult to achieve.

The composition of the present invention can also be used in combination with a steroid drug. Here, the "use in combination" means use before, simultaneously with or after a steroid treatment, including use by blending with a steroid drug, as in the below-mentioned blend.

For combined use with a steroid drug, the drug is not particularly limited as long as it is generally used for the target disease, and specific examples thereof include the steroid drugs recited above.

The composition of the present invention is also useful as a medicament, food, and the subject of application includes mammals (e.g., human, mouse, rat, hamster, rabbit, cat, dog, bovine, sheep, monkey etc.). For application to a mammal other than human, the amount of ingestion of the composition of the present invention may be appropriately adjusted according to the body weight or size of the animal.

While the administration method of the composition of the present invention as a medicament is not particularly limited, a general administration route such as oral administration, rectal administration, administration of injection, infusion can be employed.

The dosage form of the oral administration includes granule, fine granule, dusting powder, coated tablet, tablet, suppository, powder, (micro)capsule, chewable, syrup, juice, liquid, suspension, emulsion. The dosage form by injection, general dosage forms of pharmaceutical preparations such as direct intravenous injection, instillation administration, preparation prolonging the release of activity substance can be employed.

These medicaments are prepared by formulation according to a conventional method. When necessary for formulation, various pharmaceutically acceptable substances for formulation can be added. While the substance for preparation can be appropriately selected according to the dosage form of the preparation, it includes, for example, excipient, diluent, additive, disintegrant, binder, coating agent, lubricant, glidant, glazing agent, flavor, sweetener, solubilizer. Specific examples of the substance for preparation include magnesium carbonate, titanium dioxide, lactose, mannitol and other saccharides, talc, milk protein, gelatin, starch, cellulose and derivatives thereof, animal and vegetable oil, polyethylene glycol, and solvent, such as sterile water and monovalent or polyvalent alcohol (e.g., glycerol).

While the dose of the medicament of the present invention varies depending on the age, body weight or pathology of target patients, or dosage form, administration method and the like of the medicament, it is 0.005 g/kg body weight - 5 g/kg body weight of isoleucine, 0.01 g/kg body weight - 10 g/kg body weight of leucine and 0.005 g/kg body weight - 5 g/kg body weight of valine for an adult per day.

For a general adult, the dose is preferably 0.01 g/kg body weight - 1 g/kg body weight of isoleucine, 0.02 g/kg body weight - 2 g/kg body weight of leucine and 0.01 g/kg body weight - 1 g/kg body weight of valine, and more preferably, 0.02 g/kg body weight - 0.2 g/kg body weight of isoleucine, 0.04 g/kg body weight - 0.4 g/kg body weight of leucine and 0.02 g/kg body weight - 0.2 g/kg body weight of valine for an adult per day. The total amount of amino acids is preferably about 0.01 g/kg body weight - 2 g/kg body weight per day. The above-mentioned daily dose can be administered at once or in several portions. The timing of the administration is not particularly limited and may be, for example, before meal, after meal or between meals. Also, the dosing period is not particularly limited.

When the dose (amount to be ingested) of branched chain amino acids, which are the active ingredients of the medicament of the present invention, is calculated, the amount of the active ingredients of the medicament to be used for the purpose of treatment, prophylaxis of target diseases in the present invention is determined by the aforementioned calculation method. When branched chain amino acids are ingested or administered for different purposes, for example, to satisfy the need in ordinary eating habits or treatment of other diseases, such branched chain amino acids do not need to be included in the aforementioned calculation.

For example, the daily amount of branched chain amino acids to be ingested from ordinary eating habits does not need to be excluded from the calculation of the aforementioned daily dose of the active ingredients in the present invention.

Isoleucine, leucine and valine, which are the active ingredients of the medicament of the present invention, may be contained in a preparation individually or in any combination, or all may be contained in one kind of preparation. For administration after individual processing into preparations, the administration routes and the administration dosage forms thereof may be the same or different, and the timing of the administration may be simultaneous or separate. Therefore, the dosage form, timing of administration, administration route can be appropriately determined based on the kind of medicaments to be concurrently used and the effect thereof. That is, the medicament of the present invention may be a preparation simultaneously containing plural branched chain amino acids, or take the form of concomitant drugs prepared separately and used in combination. The medicament of the present invention encompasses all these medicament forms. Particularly, a dosage form containing all branched chain amino acids in a single preparation is preferable, since it can be administered conveniently.

In the present invention, the "weight ratio" means a ratio of the weights of respective ingredients in the preparation. For example, when respective active ingredients of isoleucine, leucine and valine are contained in a single preparation, it means a ratio of individual contents, and when each of the active ingredients alone, or any combination thereof is/are contained in plural preparations, it means a ratio of the weight of each active ingredient contained in each preparation.

In the present invention, the ratio of actual dose shows a ratio of a single dose or a daily dose of each active ingredient per one subject of administration (i.e., patient). For example, when each active ingredient of isoleucine, leucine and valine is contained in a single preparation and administered to a subject of administration, the weight ratio corresponds to the dose ratio. When each active ingredient is contained separately or in any combination thereof in plural preparations and administered, the weight ratio corresponds to a ratio of the total amount of each active ingredient in each preparation administered at one time or in one day.

In addition, the composition of the present invention can also be blended with a steroid drug to give a blend for the prophylaxis or treatment of muscular atrophy associated with administration of the steroid drug. When the composition of the present invention is to be blended with a steroid drug, the mixing ratio of the composition of the present invention and the steroid drug is generally within the range of 1:0.1 - 1,000,000, particularly preferably 1:1 - 1,000, in weight ratio.

Furthermore, the composition of the present invention can also be conveniently used in the form of a food. When the composition is used as a food, any meal form can be employed as long as it is a general one containing the active ingredients of the food of the present invention, i.e., branched chain amino acids. For example, a suitable flavor may be added to give a drink, such as beverage and powder drink mix. Specifically, for example, the composition can be added to juice, milk, confectionery, jelly, yogurt, candy and served for eating and drinking.

In addition, such food can also be provided as food with health claims or dietary supplement. The food with health claims also includes food for specified health uses, food with nutrient function claims. The food for specified health uses is a food permitted to indicate that it is expected to achieve a particular health object, such as suppression of muscular atrophy, improvement or suppression of side effect symptoms associated with a steroid treatment. The food with nutrient function claims is also a food permitted to indicate function of nutrition components when the amount of the nutrition components contained in the advisable daily amount of ingestion satisfies the upper and lower limits of the standard level set by the Japanese government. The dietary supplement includes what is called nutrition supplement, health supplement. In the present invention, the food for specified health uses includes a food with an indication that it is used for suppression of muscular atrophy, improvement or suppression of side effect symptoms associated with a steroid treatment, and further, a food enclosing, in a package, a written document (i.e., insert) describing that the food is used for such uses.

Furthermore, the composition of the present invention can be utilized as a concentrated liquid diet or food supplement. For use as a dietary supplement, for example, it can be formed into tablet, capsule, powder, granule, suspension, chewable, syrup. The food supplement in the present specification includes, in addition to those taken as food, those taken for the purpose of supplementing nutrition, which include nutritional supplement, supplement. The dietary supplement in the present invention can also include a subset of food with health claims.

In the present specification, the "food supplement" refers to one taken to aide the nutrition in addition to those ingested as food, and includes nutritional supplement, supplement.

In the present invention, the "concentrated liquid diet" is a total nutrition food (liquid food) designed based on the essential amount of nutrition per day and controlled to a concentration of about 1 kcal/ml, wherein qualitative constitution of each nutrition is sufficiently considered so that long-term sole ingestion of the food will not cause marked shortage or excess of nutrition.

When the composition is ingested as a food, the amount of ingestion varies depending on the symptom, age or body weight of subject of ingestion, or the form or ingestion method of food and the like. A desired amount is 0.005 g/kg body weight - 5 g/kg body weight of isoleucine, 0.01 g/kg body weight - 10 g/kg body weight of leucine and 0.005 g/kg body weight - 5 g/kg body weight of valine for an adult per day.

For a general adult, the amount is preferably 0.01 g/kg body weight - 1 g/kg body weight of isoleucine, 0.02 g/kg body weight - 2 g/kg body weight of leucine and 0.01 g/kg body weight - 1 g/kg body weight of valine, and more preferably, 0.02 g/kg body weight - 0.2 g/kg body weight of isoleucine, 0.04 g/kg body weight - 0.4 g/kg body weight of leucine and 0.02 g/kg body weight - 0.2 g/kg body weight of valine for an adult per day, and the total amount of amino acids is preferably about 0.01 g/kg body weight - 2 g/kg body weight per day. The above-mentioned daily amount of the food of the present invention can be ingested at once or in several portions. The form, ingestion method, ingestion period of the food are not particularly limited.

Isoleucine, leucine and valine have been widely used in the fields of medicaments and food, and their safety has been established. For example, acute toxicity (LD₅₀) of a pharmaceutical preparation containing these amino acids at a weight ratio of 1:2:1.2 is not less than 10 g/kg even when orally administered to mice.

Another embodiment of the present invention is use of isoleucine, leucine and valine for the production of the composition for suppressing muscular atrophy-related gene expression or the composition for improving or suppressing side effects in a steroid treatment of the present invention. Preferable scope of the composition is the same as that mentioned above.

A still another embodiment is a method for use in suppressing muscular atrophy-related gene expression or a method for use in improving or suppressing side effects in a steroid treatment, comprising administering effective amounts of isoleucine, leucine and valine to a subject of administration. Preferable range of effective amounts of the active ingredients are the same as those mentioned above.

A yet another embodiment of the present invention is a commercial package containing a written matter stating that a composition comprising three kinds of branched chain amino acids of isoleucine, leucine and valine as active ingredients can or should be used for suppressing muscular atrophy, or improving or suppressing a side effect in a steroid treatment.

The present invention is explained in more detail by referring to the following Examples, which provide mere exemplification of the present invention and do not limit the scope of the present invention.

### [Examples]

### (Example 1) Muscular atrophy prophylactic effect of BCAA in dexamethasone administered rat

Dexamethasone (600 µg/kg) was intraperitoneally administered to rats (SD; 10-11-week-old) for 5 consecutive days. An isoleucine, leucine and valine (weight ratio 1:2:1.2) blend (BCAA) administration group was orally administered with 0.75 g/kg of BCAA above simultaneously for 5 consecutive days. A vehicle group was orally administered with distilled water consecutively in the same manner.

On day 5, the rats were autopsied and analyzed for muscular strength function evaluation, blood glucose level and plasma insulin level using a feed intake, body weight profile, muscle weight, grip strength measurement apparatus.

As the control group, pair-feeding normal rats were used.

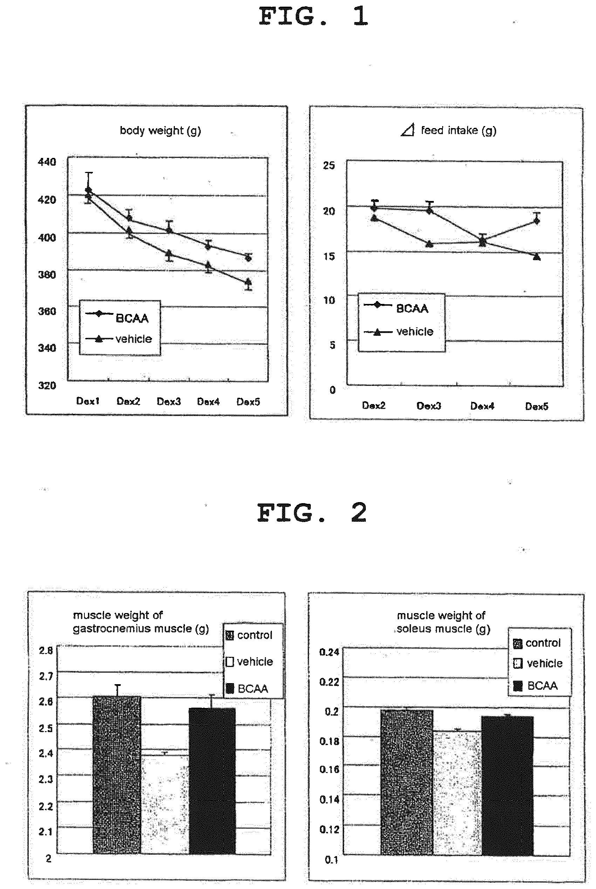

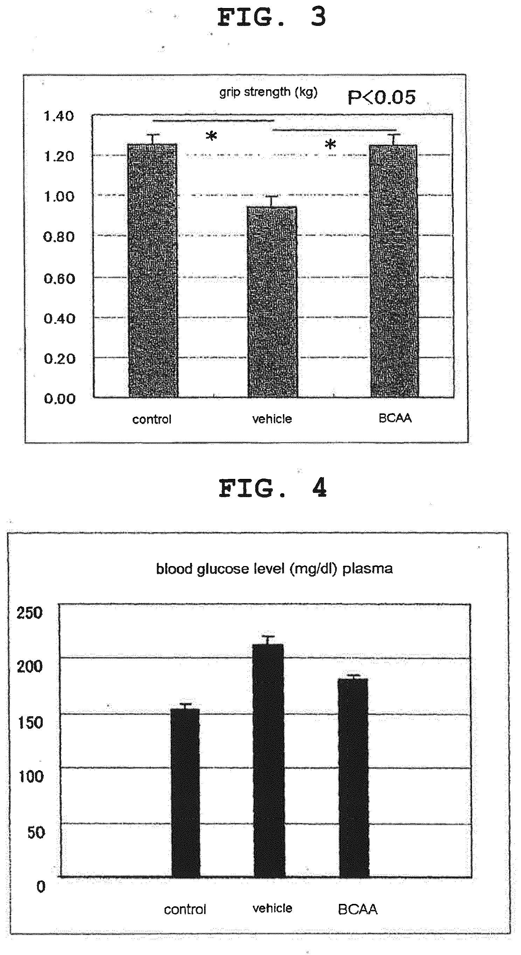

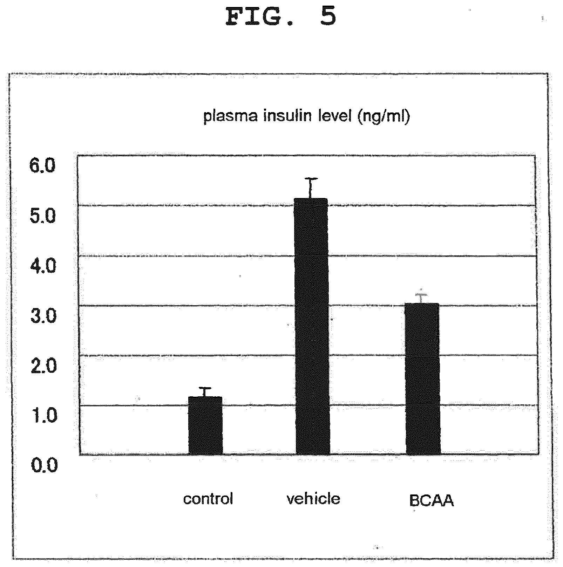

By comparison to the vehicle group, the BCAA administration group showed suppression of body weight loss and decrease in feed intake (Fig. 1). Decrease in muscle weight was also suppressed (Fig. 2). In addition, the grip strength was significantly high in the BCAA administration group, and improvement of muscle function was observed (Fig. 3). Although dexamethasone administration increased both the blood glucose level and insulin level, the increase was corrected by the administration of BCAA (Figs. 4 and 5).

### (Example 2) Muscular atrophy treatment effect of BCAA in dexamethasone administered rat

Dexamethasone (600 µg/kg) was intraperitoneally administered to rats (SD; 10-11-week-old) for 5 consecutive days to induce muscular atrophy. On day 6 and day 7 from the termination of dexamethasone administration, BCAA (0.75 g/kg) was orally administered and the treatment effect on muscular atrophy was examined. The vehicle group was orally administered with distilled water consecutively in the same manner.

On day 6 and day 7, the rats were autopsied and analyzed for muscle weight. R1 and R2 are day 1 and day 2 of the recovery period (recover; R), respectively, and correspond to day 6 and day 7 from the termination of dexamethasone administration.

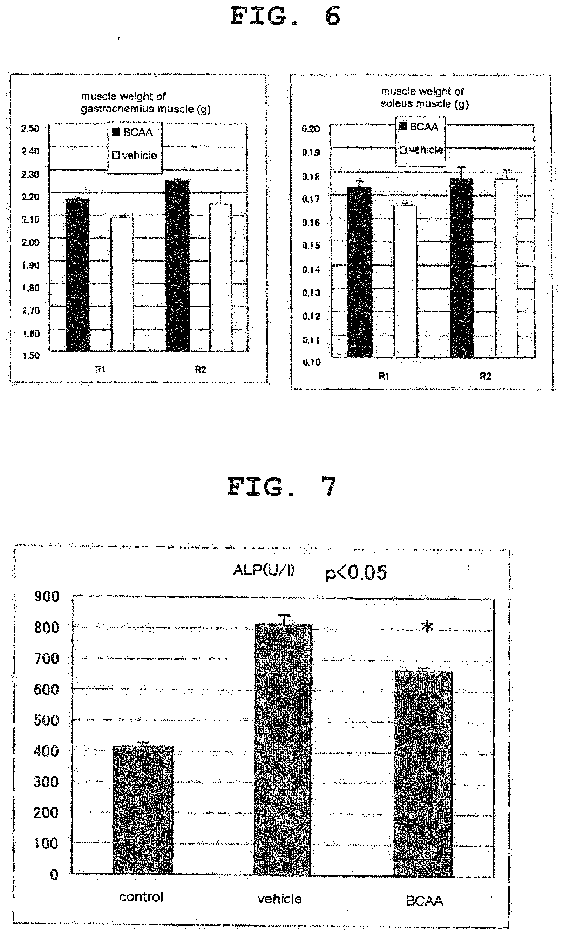

By comparison to the vehicle group, the BCAA administration group showed an early recovery of muscle weight (Fig. 6).

### (Example 3) Osteoporosis prophylactic effect of BCAA in dexamethasone administered rat

Dexamethasone (600 µg/kg) was intraperitoneally administered to rats (SD; 10-11-week-old) for consecutive 1.5 months. An isoleucine, leucine and valine (weight ratio 9:7:6) blend (BCAA) administration group was orally administered with 0.75 g/kg of BCAA above simultaneously for 1.5 consecutive months. A vehicle group was orally administered with distilled water consecutively in the same manner.

At 1.5 months, the rats were autopsied and analyzed for plasma ALP level. It is known that the plasma ALP shows a high value when bone metabolism turnover is high.

By comparison to the vehicle group, the BCAA administration group showed low plasma ALP value (Fig. 7).

### (Example 4) Muscular atrophy prophylactic effect of branched chain amino acid in dexamethasone administered rat

Dexamethasone (600 µg/kg) was intraperitoneally administered to rats (SD; 10-11-week-old) for consecutive 5 days to induce muscular atrophy. An isoleucine, leucine and valine (weight ratio 1:2:1.2) blend (BCAA) administration group (indicated as BCAA in Figures and hereinafter to be indicated as BCAA administration group) was orally administered with 0.75 g/kg of BCAA above simultaneously for 5 consecutive days. A vehicle group was orally administered with distilled water consecutively in the same manner. On day 5, the rats were autopsied and measured for feed intake, body weight profile and muscle weight and the expression amount of intramuscular atrophy gene was analyzed by a method known per se.

To be specific, cDNA was synthesized using, as a template, RNA extracted from skeletal muscle using ISOGEN (NIPPON GENE), and SuperScript III (Invitrogen). cDNA (2.5 ng), primers of Atrogin-1 and MuRF-1 (see the following Table 1) and power cyber green (Applied Biosystems) were mixed, and the expression amounts of muscular atrophy-related genes (Atrogin-1, MuRF-1, Myostatin, FOXO1, FOXO3a, FOXO4, REDD1 and KLF15) present in test samples were relatively and quantitatively analyzed by real-time RT-PCR method with that of the control group as 1.

As the control group, pair-feeding normal rats were used.

**[Table 1]**

| gene name | 5'-primer | 3'-primer |
|---|---|---|
| atrogin-1 | AGCGCTTCTTGGATGAGAAA (SEQ ID NO:1) | TCTTGGCAACATCGTAG (SEQ ID NO:2) |
| MuRF-1 | CCAGGTGAAGGAGGAACTGA (SEQ ID NO:3) | CTCCTGCTCCTGAGTGATCC (SEQ ID NO:4) |
| Myostatin | ACGCTACCACGGAAACAATC (SEQ ID NO:5) | GGAGTCTTGACGGCTCTCAG (SEQ ID NO:6) |
| Foxo 1 | CGGCCAATCCAGCATGAGCCC (SEQ ID NO:7) | GTGGGAGGAGAGTCAGAAGTC (SEQ ID NO:8) |
| Foxo 3a | GCTCCACCACCAGCACCAAACC (SEQ ID NO:9) | CGAGAGGGTTTGCATAGACTGGC (SEQ ID NO:10) |
| Foxo 4 | GOCATGACAGAATGCCTCAGGATC (SEQ ID NO:11) | GGCTCAAAGTTGAAGTCCAGTCCC (SEQ ID NO:12) |
| REDD1 | TAGTGCCCACCTTTCAGTTG (SEQ ID NO:13) | GTCAGGGACTGGCTGTAACC (SEQ ID NO:14) |
| KLF15 | CTGCAGCCAAGATGTACACCAA (SEQ ID NO:15) | TCATCTGAGCGTGAAAACCTC (SEQ ID NO:16) |

It is known that dexamethasone administration to rat decreases body weight and feed intake, and the expression of atrogin-1, MuRF-1 and Myostatin, which are proteasomal degradation inducing genes, is induced to develop muscular atrophy, which reduces the muscle amount (see Endocrinology 146: 1789-1797 (2005), Am. J. Physiol. Endocrinol. Metab. 287: E591-E601 (2004)).

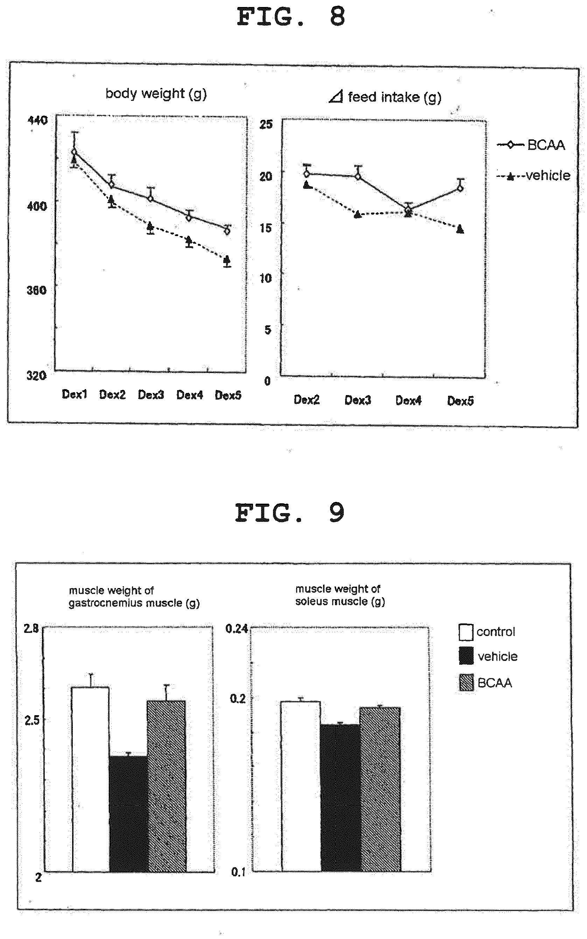

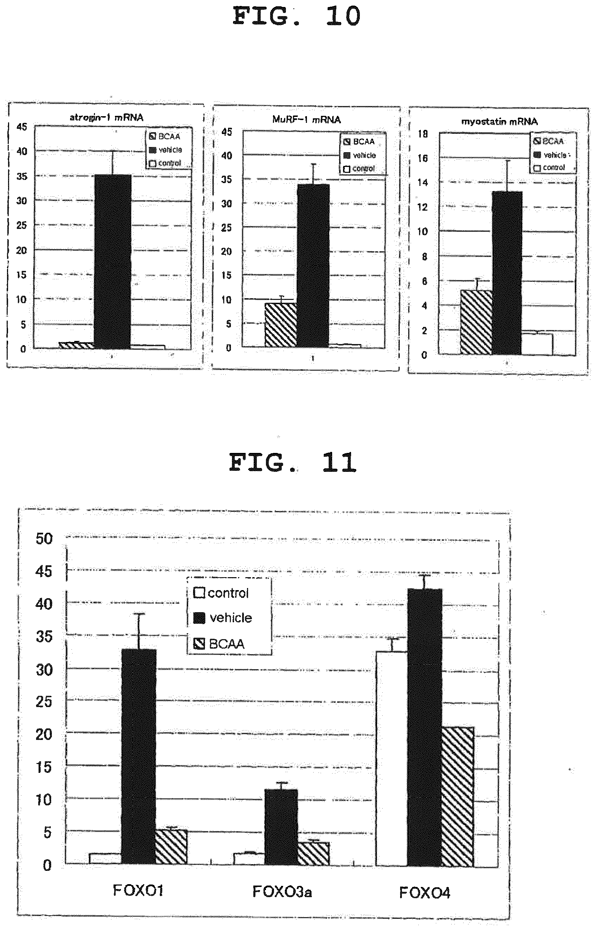

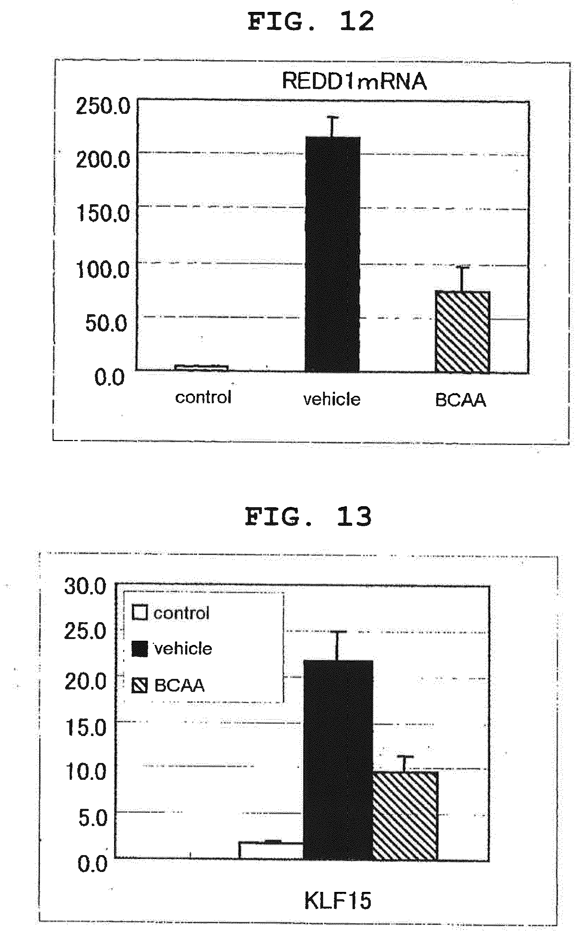

In the vehicle group, decrease in the body weight and feed intake was observed. However, such decrease was suppressed in the BCAA administration group (Fig. 8). In the BCAA administration group, decrease in the muscle weight was suppressed as compared to the vehicle group (Fig. 9). In the BCAA administration group, moreover, expressions of muscular atrophy-related genes atrogin-1 and MuRF-1 and Myostatin, a negative growth factor of muscle, as well as FOXO1, FOXO3a, FOXO4, REDD1 and KLF15 genes, which are atrophy-related genes, were also suppressed (Figs. 10 - 13).

### (Example 5) Muscular atrophy treatment effect of BCAA in dexamethasone administration rat

Dexamethasone (600 µg/kg) was intraperitoneally administered to rats (SD; 10-11-week-old) for consecutive 5 days to induce muscular atrophy. On day 6 and day 7 from the termination of dexamethasone administration, an isoleucine, leucine and valine (weight ratio 1:2:1.2) blend (0.75 g/kg) was orally administered and the treatment effect on muscular atrophy was examined. The vehicle group was orally administered with distilled water consecutively in the same manner. On day 6 and day 7, the rats were autopsied and analyzed for feed intake, body weight, muscle weight, muscular atrophy gene expression amount. R1 and R2 are day 1 and day 2 of the recovery period (recover; R), respectively, and correspond to day 6 and day 7 from the termination of dexamethasone administration.

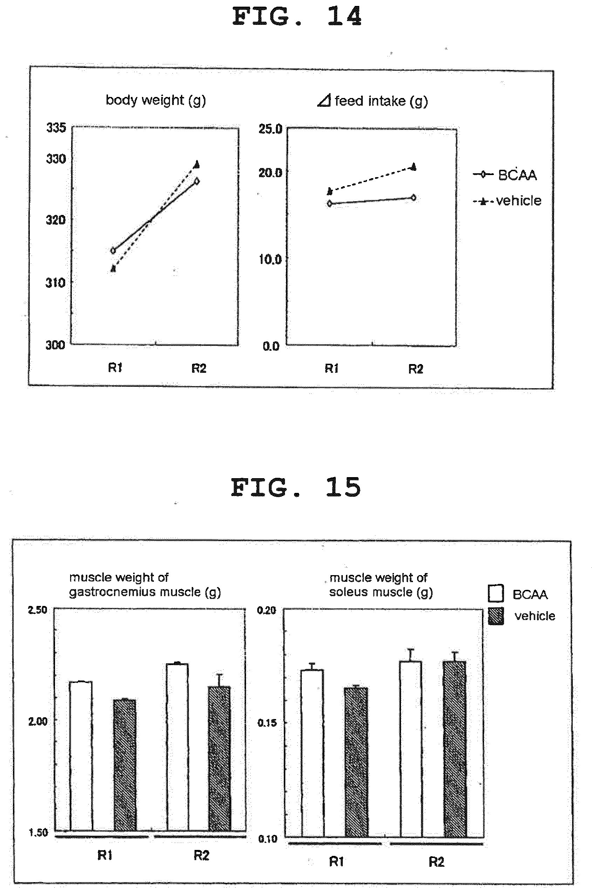

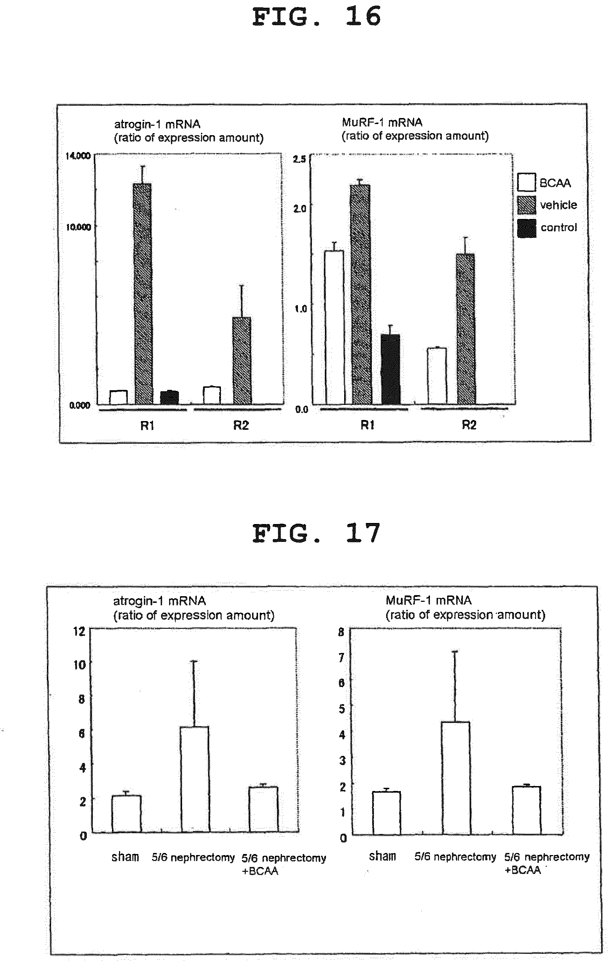

By comparison to the vehicle group, although the BCAA administration group showed low level of recovery of body weight loss and decrease in feed intake (Fig. 14), it showed a high recovery level of the muscle weight (Fig. 15). In addition, the expression amount of muscular atrophy gene was quantitatively analyzed by the above-mentioned real-time PCR method. As a result, the BCAA administration group also remarkably suppressed expression of muscular atrophy genes atrogin-1 and MuRF-1 (Fig. 16).

From the foregoing results, it has been clarified that the recovery of muscle weight by BCAA administration is attributable to the suppression of muscular atrophy resulting from the suppression of muscular atrophy gene expression, rather than to an increase in the feed intake.

### (Example 6) Suppression of muscular atrophy gene expression of BCAA in 5/6 nephrectomy rat

Rats (WKY; 7-9-week-old) were subjected to 5/6 nephrectomy to prepare renal failure model rats. The expression of muscular atrophy gene was analyzed by the above-mentioned real-time PCR method in three groups of the aforementioned model rats: groups with or without BCAA administration and a group with sham operation alone (sham). In the renal failure model rat group free of BCAA administration, the expression of muscular atrophy genes atrogin-1 and MuRF-1 increased as compared to the sham group. In contrast, the BCAA administration group suppressed the expression of atrogin-1 and MuRF-1 almost to the level of the sham group (Fig. 17). In Fig. 17, an average of the relative value of each group was plotted with the value of one case in the sham group as 1.0.

From the foregoing results, it has been clarified that BCAA has a suppressive effect even on an increase in the muscular atrophy gene expression due to renal failure.

This application is based on a patent application Nos. 2006-335057 and No. 2006-335059 filed in Japan.

## Claims

1. A composition for use in improving or suppressing a side effect in a steroid treatment, comprising isoleucine, leucine and valine as active ingredients wherein the side effect is at least one kind selected from the group consisting of muscular atrophy, decreased muscle function, muscular pain, arthritic pain, impaired glucose tolerance, loss of appetite, body weight loss, decreased bone metabolism, impaired immunity and fatigability.

2. The composition for use according to claim 1, wherein the side effect is impaired glucose tolerance and/or decreased bone metabolism.

3. The composition for use according to claim 1, wherein muscular atrophy shows promoted expression of muscular atrophy-related gene.

4. A composition for use in suppressing muscular atrophy associated with promoted expression of muscular atrophy-related gene, comprising isoleucine, leucine and valine as active ingredients, wherein the promoted expression is associated with renal failure, diabetes, lung failure, liver failure or cardiac failure.

5. The composition for use according to claim 4 wherein the muscular atrophy-related gene is at least one kind selected from the group consisting of atrogin-1 gene, MuRF-1 gene, myostatin gene, FOXO1 gene, FOXO3a gene, FOXO4 gene, REDD1 gene and KLF15 gene.

6. The composition for use according to any one of claims 1 to 5, comprising not less than 1 g of isoleucine, leucine and valine in one-time ingestion.

7. The composition for use according to any one of claims 1 to 6, wherein a weight ratio of isoleucine, leucine and valine is 1:1.5 - 2.5:0.8 - 1.7.

8. The composition for use according to any one of claims 1 to 7, for use in combination with a steroid drug.

9. The composition for use according to any one of claims 1 to 7, further comprising a steroid drug.

10. The composition for use according to any one of claims 1 to 9, which is a medicament.

11. The composition for use according to any one of claims 1 to 9, which is a food, or dietary supplement.

12. The composition for use according to claim 11, wherein the food is a concentrated liquid diet.

13. Use of isoleucine, leucine and valine for the production of a composition for use according to any one of claims 1 to 12.

## Patentansprüche

1. Zusammensetzung zur Verwendung bei der Verbesserung oder Unterdrückung von Nebenwirkungen einer Steroidtherapie, umfassend Isoleucin, Leucin und Valin als aktive Inhaltsstoffe, wobei die Nebenwirkung von einer Art ist, die ausgewählt ist aus der Gruppe bestehend aus Muskelschwund, verringerter Muskelfunktion, Muskelschmerzen, arthritischen Schmerzen, verschlechterter Glukosetoleranz, Appetitverlust, Körpergewichtsverlust, verringertem Knochenmetabolismus, verschlechterter Immunabwehr und Müdigkeit.

2. Zusammensetzung zur Verwendung nach Anspruch 1, wobei die Nebenwirkung verschlechterte Glukosetoleranz und/oder verringerter Knochenmetabolismus ist.

3. Zusammensetzung zur Verwendung nach Anspruch 1, wobei der Muskelschwund eine erhöhte Expression von Muskelschwund-bezogenen Genen zeigt.

4. Zusammensetzung zur Verwendung in der Unterdrückung von Muskelschund im Zusammenhang mit der erhöhten Expression von Muskelschwund-bezogenen Genen, umfassend Interleucin, Leucin und Valin als aktive Inhaltsstoffe, wobei die erhöhte Expression in Zusammenhang steht mit Nierenversagen, Diabetes, Lungenversagen, Leberversagen oder Herzversagen.

5. Zusammensetzung zur Verwendung nach Anspruch 4, wobei das Muskelschwund-bezogene Gen zumindest eine Art hat, die ausgewählt ist aus der Gruppe bestehend aus Atrogin-1 Gen, MuRF-1 Gen, Myostatin-Gen, FOXO1 Gen, FOXO3a Gen, FOXO4 Gen, REDD1 Gen und KLF15 Gen.

6. Zusammensetzung zur Verwendung nach einem der Ansprüche 1 bis 5, umfassend nicht weniger als 1 g Isoleucin, Leucin und Valin in der einmaligen Einnahme.

7. Zusammensetzung zur Verwendung nach einem der Ansprüche 1 bis 6, wobei ein Gewichtsverhältnis von Isoleucin, Leucin und Valin 1:1,5 - 2,5:0,8 - 1,7 beträgt.

8. Zusammensetzung zur Verwendung nach einem der Ansprüche 1 bis 7 zur Verwendung in Kombination mit einem Steroidmedikament.

9. Zusammensetzung zur Verwendung nach einem der Ansprüche 1 bis 7, ferner umfassend ein Steroidmedikament.

10. Zusammensetzung zur Verwendung nach einem der Ansprüche 1 bis 9 als ein Medikament.

11. Zusammensetzung zur Verwendung nach einem der Ansprüche 1 bis 9 als ein Nahrungsmittel oder Nahrungsergänzungsmittel.

12. Zusammensetzung zur Verwendung nach Anspruch 11, wobei das Nahrungsmittel eine konzentrierte Flüssignahrung ist.

13. Verwendung von Isoleucin, Leucin und Valin zur Herstellung einer Zusammensetzung zur Verwendung nach einem der Ansprüche 1 bis 12.

## Revendications

1. Composition destinée à être utilisée pour améliorer ou supprimer un effet secondaire dans un traitement basé sur des stéroïdes, comprenant de l'isoleucine, de la leucine et de la valine à titre de principes actifs dans laquelle l'effet secondaire est au moins d'un type choisi dans le groupe constitué par l'atrophie musculaire, une fonction musculaire diminuée, la douleur musculaire, la douleur arthritique, une tolérance altérée au glucose, la perte d'appétit, la perte de poids corporel, un métabolisme osseux diminué, une immunité altérée et la fatigabilité.

2. Composition pour son utilisation selon la revendication 1, dans laquelle l'effet secondaire est une tolérance altérée au glucose et/ou un métabolisme osseux diminué.

3. Composition pour son utilisation selon la revendication 1, dans laquelle l'atrophie musculaire montre une expression favorisée du gène lié à l'atrophie musculaire.

4. Composition pour son utilisation dans la suppression de l'atrophie musculaire associée à une expression favorisée du gène lié à l'atrophie musculaire, comprenant de l'isoleucine, de la leucine et de la valine à titre de principes actifs, dans laquelle l'expression favorisée est associée à l'insuffisance rénale, au diabète, à l'insuffisance pulmonaire, à l'insuffisance hépatique ou à l'insuffisance cardiaque.

5. Composition pour son utilisation selon la revendication 4 dans laquelle le gène lié à l'atrophie musculaire est au moins d'un type choisi dans le groupe constitué par le gène de l'atrogine-1, le gène MuRF-1, le gène de la myostatine, le gène FOX01, le gène FOX03a, le gène FOX04, le gène REDD1 et le gène KLF15.

6. Composition pour son utilisation selon l'une quelconque des revendications 1 à 5, ne comprenant pas moins de 1 g d'isoleucine, de leucine et de valine par prise ingérée.

7. Composition pour son utilisation selon l'une quelconque des revendications 1 à 6, dans laquelle un rapport en poids isoleucine:leucine:valine est de 1:1,5-2,5:0,8-1,7.

8. Composition pour son utilisation selon l'une quelconque des revendications 1 à 7, destinée à être utilisée en association avec un médicament stéroïdien.

9. Composition pour son utilisation selon l'une quelconque des revendications 1 à 7, comprenant en outre un médicament stéroïdien.

10. Composition pour son utilisation selon l'une quelconque des revendications 1 à 9, qui est un médicament.

11. Composition pour son utilisation selon l'une quelconque des revendications 1 à 9, qui est un aliment, ou un complément alimentaire.

12. Composition pour son utilisation selon la revendication 11, dans laquelle l'aliment est un régime liquide concentré.

13. Utilisation de l'isoleucine, de la leucine et de la valine pour la production d'une composition destinée à être utilisée selon l'une quelconque des revendications 1 à 12.
